## Europäisches Patentamt
## European Patent Office
## Office européen des brevets

(11) Veröffentlichungsnummer: **0 131 927**
**B1**

(12) **EUROPÄISCHE PATENTSCHRIFT**

(45) Veröffentlichungstag der Patentschrift:
**04.10.89**

(21) Anmeldenummer: **84108234.0**

(22) Anmeldetag: **12.07.84**

(51) Int. Cl.⁴: **A 61 K 31/34**, A 61 K 47/00

(54) Pharmazeutische Zubereitung enthaltend Isosorbiddinitrat.

(30) Priorität: **14.07.83 DE 3325466**

(43) Veröffentlichungstag der Anmeldung:
**23.01.85 Patentblatt 85/4**

(45) Bekanntmachung des Hinweises auf die Patenterteilung:
**04.10.89 Patentblatt 89/40**

(84) Benannte Vertragsstaaten:
**AT CH DE FR LI**

(56) Entgegenhaltungen:
**DE-A-1 719 443**
**LU-A-81 902**

(73) Patentinhaber: **Heinrich Mack Nachf., Postfach 140, D-7918 Illertissen (DE)**

(72) Erfinder: **Fries, Walter, Einsteinring 25, D-7918 Illertissen (DE)**

(74) Vertreter: **Lederer, Franz, Dr., Van der Werth, Lederer & Riederer Patentanwälte Lucile- Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft pharmazeutische Zubereitungen in Salbenform, enthaltend Isosorbiddinitrat (ISDN).

ISDN ist ein bekannter pharmakologischer Wirkstoff, der insbesondere zur Bekämpfung koronarer Herzkrankheiten wie beispielsweise Angina pectoris, eingesetzt wird. Seit längerer Zeit bemüht man sich, die Darreichungsform des Wirkstoffes zu verbessern, um damit seine Verabreichung zu erleichtern und seine Wirksamkeit zu verbessern. In diesem Zusammenhang haben topische Anwendungsformen zunehmend an Interesse gewonnen und die erfindungsgemäße Zusammensetzung ist für topische Anwendung bestimmt.

In der US-A-4 112 115 wird die Anwendung von ISDN mit ein Träger offenbart, wobei das ISDN in Form einer echten Lösung in einer oder mehreren Phasen des Trägers vorliegt. Mit diesen Lösungen kann keine Langzeitwirkung erzielt werden.

Die DE-C-2 623 800 offenbart eine übersättigte wässrige ISDN-Lösung, die insbesondere für parenterale Verabreichung bestimmt ist und frei von ISDN-Bodenkörper sein muß.

Die DE-A-1 719 443 beschreibt Suspensions-Aerosole, die unter anderem auch Isosorbiddinitrat enthalten können. Im Vordergrund steht dabei die Frage der Stabilität im Hinblick auf eine Sedimentation.

Eine topische Anwendungsform für ISDN wird in der DE-A-3 200 369 beschrieben, die ein Präparat, bestehend aus einem flexiblen Träger und einem Basismaterial aus einem Polymer, welches das ISDN enthält, offenbart, wobei das Präparat auf die Haut aufgelegt wird.

Die DE-A-2 924 005 beschreibt eine pharmazeutische Zubereitung salbenförmiger Konsistenz enthaltend ISDN als Wirkstoff, einen Salbenkonsistenzgeber, einen Emulgator und Wasser sowie ein Lösungsmittel für ISDN, welches mindestens 5, vorzugsweise mindestens 10 Gew.-% ISDN (bezogen auf das Lösungsmittel) löst und mit Wasser mischbar ist. Diese bekannte Zubereitung ist für eine Applikation auf die Haut geeignet und besitzt eine gute Langzeitwirkung, da ein Teil des ISDN in gelöster, ein weiterer Anteil des ISDN in ungelöster, feinkristalliner Form vorliegt. Die ungelösten ISDN-Teilchen sollen eine Länge von höchstens 100 und eine Breite von höchstens 20 μm besitzen, um ein rasches Nachlösen der ISDN-Kristalle auf der Haut sicherzustellen. Als Lösungsmittel in diesen bekannten Zubereitungen werden gewisse Fettsäureester empfohlen.

Es wurde als Nachteil empfunden, daß in diesen salbenförmig Präparaten bei der Lagerung ein Kristallwachstum stattfindet was sich auf das Nachlösen der ISDN-Kristalle auf der Haut und somit auf die Höhe und Reproduzierbarkeit der Blutspiegelwerte ungünstig auswirkt.

Aufgabe der vorliegenden Erfindung ist es daher, eine pharmazeutische Zubereitung in Form einer Salbe enthaltend ISDN als Wirkstoff in zumindest teilweise ungelöster, feinkristalliner Form zur Verfügung zu stellen, die auch bei ungünstigen Lagerbedingungen ein vermindertes Kristallwachstum zeigt und einen hohen und lang andauernden Blutspiegel bewirkt.

Diese Aufgabe wird durch eine pharmazeutische Zubereitung in Form einer Salbe gelöst, wie sie in den Patentansprüchen beschrieben wird.

Es ist bereits bekannt (vgl. Hagers Handbuch der Pharm. Praxis, Springer Verlag, 1971, Seite 555 und R. Voigt: Lehrbuch der pharmazeutischen Technologie, VEB Verlag, Volk und Gesundheit, Berlin, 1973, Seite 310), daß Suspensionssalben nur dann lagerstabile Systeme darstellen, wenn der suspendierte Arzneistoff in der verwendeten Salbengrundlage vollkommen unlöslich ist. Da in der Praxis aber meist, und so auch im vorliegenden Fall, die inkorporierten Arzneistoffe in der Salbengrundlage nicht vollständig unlöslich sind, muß immer mit Kristallwachstum gerechnet werden. Es ist umso mehr überraschend, daß erfindungsgemäß sowohl eine praktisch vollständige Unterdrückung des Kristallwachstums erzielt wird, als auch sogar noch eine Wirkungssteigerung, die in einem erhöhten Blutspiegel an der Wirksubstanz zum Ausdruck kommt.

Die in den erfindungsgemäßen Zubereitungen verwendeten Lösungsmittel besitzen eine Löslichkeit für ISDN von unter 5 Gew.-% (bezogen auf das Lösungsmittel). Vorzugsweise beträgt ihre Löslichkeit für ISDN 1 - 3 Gew.-% (Wasser besitzt eine Löslichkeit für ISDN von etwa 1,5 Gew.-%).

Die erfindungsgemäßen Zubereitungen enthalten 5 - 15, vorzugsweise 8 - 12 Gew.-% ISDN. Die Menge an ISDN ist so, daß immer ungelöstes ISDN mit vorliegt.

Das ungelöste ISDN liegt in fein-kristalliner Form vor in einer Teilchengröße unter 100 μm, vorzugsweise mit einer mittleren Teilchengröße von 5 - 40 μm, und besonders bevorzugt mit einer mittleren Teilchengröße von 10 μm. Die Teilchengröße wird dabei gemessen als Durchmesser des Kreises, der mit der Projektionsfläche des jeweiligen Teilchens flächengleich ist.

Als Lösungsmittel werden in den erfindungsgemäßen Zubereitungen vorzugsweise Ölsäureester verschiedener Fettalkohole ($C_{10}$ - $C_{18}$) wie z. B. Decylalkohol, Laurinalkohol usw. eingesetzt. Insbesondere wird Decyloleat bevorzugt. Auch Gemische von Fettalkoholestern sind geeignet.

Als weitere Lösungsmittel eignen sich nichtionogene Sorbitanfettsäureester. Geeignet erscheinen beispielsweise flüssige Sorbitanmono-, Sorbitantri- und Sorbitansesquiester mit gesättigten und ungesättigten Fettsäuren, die 10 - 18 Kohlenstoffatome enthalten, wie z. B. Caprinsäure, Laurinsäure, Myristinsäure, Palmitinsäure, Stearinsäure, Palmitoleinsäure, Ölsäure, Linolsäure. Besonders bevorzugt wird Sorbitansesquioleat.

In einer bevorzugten Ausführungsform der Erfindung werden Gemische von Fettalkohol-Ölsäure-Ester und Sorbitanfettsäureester verwendet. In diesem Fall kann der Fettalkohol-Ölsäure-Ester in einer Menge (bezogen auf die gesamte Zusammensetzung) von 5 - 40, vorzugsweise 20 - 30 Gew.-% und der Sorbitanfettsäureester in einer Menge (bezogen auf die gesamte Zusammensetzung) von 1 - 10, vorzugsweise 1 - 5 Gew.-% eingesetzt werden.

Besonders bevorzugt wird eine Kombination von 20 - 30, insbesondere 24 Gew.-% Decyloleat und 1 - 5, insbesondere 2 Gew.-% Sorbitansesquioleat (jeweils bezogen auf die gesamte Zusammensetzung).

Die erfindungsgemäßen Zubereitungen enthalten zweckmäßigerweise noch einen emulgierenden und/oder körpergebenden Zusatz. Als solcher eignen sich beispielsweise Gemische höherer gesättigter Fettalkohole mit Fettalkoholsulfaten und Emulgatoren wie Polyglykoläthern.

Die genaue Zusammensetzung dieser Zusätze ist nicht besonders kritisch, da sie für die Unterdrückung des Kristallwachstums keinen oder nur einen sehr geringen Beitrag liefern. Solche Zusätze werden dennoch bevorzugt, beispielsweise auch um den Zubereitungen Konsistenz zu geben, wenn man die Zubereitungen in Form von Salben konfektioniert.

Besonders geeignet ist ein kolloid-disperses Gemisch von Cetylstearylalkohol, Natriumcetylstearylsulfat und einem Triglycerid mit 40 Mol Ethylenoxid oder Fettalkoholpolyglykoläther, welches unter der Bezeichnung Emulgade F im Handel ist.

Der emulgierende und/oder körpergebende Zusatz kann in eine Menge von 1 - 8, vorzugsweise 5 - 7 Gew.-% anwesend sein.

Als wesentlichen Bestandteil enthalten die erfindungsgemäße Zubereitungen schließlich noch Wasser in einer Menge von 30 - 75, vorzugsweise 40 - 60 Gew.-%. Andere übliche Zusätze wie Antioxydantien, Geruchsstoffe, Konservierungsmittel können in untergeordneten Mengen anwesend sein.

Die erfindungsgemäßen Zubereitungen werden in Form von Salben konfektioniert, die zweckmäßigerweise eine Viskosität von 20 000 - 80 000 mPa.s, vorzugsweise etwa 50 000 mPa.s (gemessen mit Hilfe eines Brookfield-Viskosimeters Typ LVT mit Spindel 3 und 1,5 U/min) aufweisen.

Eine besonders bevorzugte erfindungsgemäße Salbe besitzt folgende Zusammensetzung:

10     Gew.-% ISDN
24     Gew.-% Decyloleat
2      Gew.-% Sorbitansesquioleat
6      Gew.-% Emulgade®F
57,85  Gew.-% Wasser
 0,15  Gew.-% Sorbinsäure.

Eine solche Salbe ist besonders bequem mit einer Pumpe dosierbar. Eine geeignete Applikationsmenge für eine Applikation beträgt 0,4 - 3 g Salbe, entsprechend 40 - 300 mg ISDN, insbesondere 0,6 - 2 g Salbe, entsprechend 60 - 200 mg ISDN und besonders bevorzugt etwa 1 g Salbe, entsprechend 100 mg ISDN, die zweckmäßigerweise auf eine Hautfläche von etwa 200 cm² aufgetragen werden. Üblicherweise findet 1 Applikation pro Tag statt jedoch können in Ausnahmefällen auch zwei und mehr Applikationen erfolgen, je nach den vom Arzt zu bestimmenden Bedürfnissen.

**Beispiel**

72 g Ölsäureester des Decylalkohols, 6 g Sorbitansesquioleat und 18 g Gemisch höherer gesättigter Fettalkohole mit Fettalkoholsulfaten und Emulgatoren (Emulgade®F) wurden zum Schmelzen auf 80°C erwärmt. Diese Ölphase wurde mit 176 g auf 70°C erwärmtem demineralisiertem Wasser bei dieser Temperatur unter intensivem Rühren vereinigt. Die erhaltene Emulsion wurde auf 25°C gekühlt und in zwei Hälften geteilt. 30 g feinkristallines Isosorbiddinitrat mit einer Korngröße von unter 100 μm wurden mittels eines hochtourigen Rührers in der einen Hälfte der Creme bei Zimmertemperatur suspendiert und anschließend homogenisiert.

Zu diesem Konzentrat wurde die restliche Hälfte der Creme gegeben, wieder intensiv bei Zimmertemperatur gerührt und zum Schluß homogenisiert.

**Vergleichsversuche**

A) Eine nach obigem Beispiel hergestellte Creme wurde 7 Monate einem Temperaturschaukeltest mit folgenden Bedingungen unterzogen:
2 Tage Lagerung bei 4°C und anschließend
2 Tage Lagerung bei 40°C.

Es wurde kein Kristallwachstum festgestellt.

Zum Vergleich wurde eine Creme nach Beispiel 1 der DE-A-2 924 005 hergestellt und dem gleichen Temperaturschaukeltest unterworfen. Diese Creme zeigte unter den gleichen Bedingungen ein Kristallwachstum auf das 2,5-fache der Ausgangswerte.

B) Weitere Proben der Creme nach obigem Beispiel und nach Beispiel 1 der DE-A-2 924 005 wurden 3 Einfrier- und Auftaucyclen unterworfen (12 Stunden bei -30°C und anschließend 12 Stunden bei +25°C im 3-maligen Wechsel). Die erfindungsgemäße Cremeformulierung zeigte keine Änderung der Kristallgröße. Die Vergleichscreme zeigte ein Kristallwachstum von ca. 20 %.

C) Nach einmaliger Applikation der erfindungsgemäßen Creme nach obigem Beispiel (Dosis 100 mg ISDN) an 8 gesunden männlichen Probanden wurde durch ISDN Plasmaspiegelmessungen die Bioverfügbarkeit von ISDN aus dieser Formulierung untersucht. Die Untersuchung erfolgte über Kreuz gegen die Salbe gemäß DE-A-2 924 005 in der gleichen Dosierung als Standardpräparat.

Nach Applikation der ISDN Salbenformen wurde ein stark protrahierter Absorptionsverlauf von ISDN beobachtet: Die Maxima im mittleren Konzentrationsverlauf traten 6 Stunden (Erfindung) und 8 Stunden (Stand der Technik) nach Verabreichung auf. Die Halbwertzeiten des danach beobachteten Konzentrationsabstiegs betrugen 13,6 Stunden (Erfindung) und 15,8 Stunden (Stand der Technik). Im Zeitraum zwischen 24 und 32 Stunden nach Verabreichung wurden noch mittlere ISDN Plasmaspiegel von ca. 1 ng/ml gemessen.

Die mittleren ISDN Konzentrationen waren nach Anwendung der erfindungsgemäßen Salbe über den gesamten Zeitraum höher als nach Anwendung des Standardpräparates. 6 und 8 Stunden nach Verabreichung waren die mittleren Plasmakonzentrationen bei der erfindungsgemäßen Salbe mit 2,97 ng/ml und 2,94 ng/ml signifikant höher (p = 0,05) als die entsprechenden Konzentrationen bei der Salbe gemäß DE-A-2 924 005 (1,44 ng/ml bzw. 1,89 ng/ml).

**Patentansprüche**

1. Pharmazeutische Zubereitung in Form einer Salbe, enthaltend Isosorbiddinitrat als Wirkstoff in zumindest teilweise ungelöster, feinkristalliner Form sowie organische Lösungsmittel und Wasser sowie gegebenenfalls einen Emulgator und andere übliche Zusätze, dadurch gekennzeichnet, daß das organische Lösungsmittel eine Löslichkeit für Isosorbiddinitrat unter 5 Gew.-%, vorzugsweise unter 3 Gew.-%, besitzt.

2. Zubereitung nach Anspruch 1 dadurch gekennzeichnet, daß das organische Lösungsmittel eines oder mehrere aus der Gruppe der Ölsäureester von Fettalkoholen und nicht-ionogenen Sorbitanfettsäureester ist bzw. sind.

3. Zubereitung nach Anspruch 1 oder 2, dadurch gekennzeichnet, daß in ungelöster Form vorliegendes Isosorbiddinitrat in einer Teilchengröße unter 100 µm, vorzugsweise mit einer mittleren Teilchengröße von 5 - 40 µm, vorliegt.

4. Zubereitung nach einem der vorgehenden Ansprüche, dadurch gekennzeichnet, daß sie

5 - 15, vorzugsweise 8 - 12 Gew.-% Isosorbiddinitrat,
5 - 40, vorzugsweise 20 - 30 Gew.-% eines Fettalkohol-Ölsäure-Esters,
1 - 10, vorzugsweise 1 - 5 Gew.-% eines Sorbitanfettsäureesters,
1 - 8, vorzugsweise 5 - 7 Gew.-% eines emulgierenden und/oder körpergebenden Zusatzes,
30 - 75, vorzugsweise 40 - 60 Gew.-% Wasser,
sowie gegebenenfalls weitere Zusätze, wie Antioxydantien, Geruchsstoffe, enthält.

5. Zubereitung nach einem der vorgehenden Ansprüche, dadurch gekennzeichnet, daß sie als Fettalkohol-Ölsäure-Ester Ölsäuredecylester enthält.

6. Zubereitung nach einem der vorgehenden Ansprüche, dadurch gekennzeichnet, daß sie als Sorbitanfettsäureester Sorbitansesquioleat enthält.

7. Zubereitung nach einem der vorgehenden Ansprüche, dadurch gekennzeichnet, daß sie als emulgierenden und körpergebenden Zusatz ein Gemisch höherer gesättigter Fettalkohole mit Fettalkoholsulfaten und Emulgatoren enthält.

8. Zubereitung nach einem der vorgehenden Ansprüche enthaltend

8 - 12, vorzugsweise 10 Gew.-% Isosorbiddinitrat, welches insoweit als es in ungelöster Form vorliegt, eine mittlere Teilchengröße von 5 - 40, vorzugsweise 10 aufweist;
20 - 30, vorzugsweise 24 Gew.-% Decyloleat,
1 - 5, vorzugsweise 2 Gew.-% Sorbitansesquioleat,
5 - 7, vorzugsweise 6 Gew.-% eines emulgierenden und körpergebenden Zusatzes bestehend aus einem kolloiddispersen Gemisch von Cetylstearylalkohol, Natriumcetylstearylsulfat und einem Triglycerid mit 40 Mol Ethylenoxid oder Fettalkoholpolyglykoläther,
50 - 60, vorzugsweise 58 Gew.-% Wasser,
sowie weitere Zusätze, wie Sorbinsäure, in Mengen unterhalb von 1 Gew.-%.

**Claims**

1. Pharmaceutical formulation in ointment form containing isosorbide dinitrate as the active compound in at least partly undissolved, finely crystalline form and organic solvents and water and optionally an emulsifier and other usual additives, characterized in that the organic solvent has a solubility for isosorbide dinitrate of less than 5 % by weight, preferably less than 3 % by weight.

2. Formulation according to claim 1, characterized in that the organic solvent is one or more of the group of the fatty alcohol oleates and nonionic sorbitan fatty acid esters.

3. Formulation according to claim 1 or 2, characterized in that the isosorbide dinitrate present in undissolved form has a particle size of less than 100 µm, preferably an average particle size of 5 - 40 µm.

4. Formulation according to one of the preceding claims, characterized in that it contains:

5 - 15, preferably 8 - 12 % by weight of isosorbide dinitrate,
5 - 40, preferably 20 - 30 % by weight of a fatty alcohol oleate,
1 - 10, preferably 1 - 5 % by weight of a sorbitan fatty acid ester,
1 - 8, preferably 5 - 7 % by weight of an emulsifying additive and/or additive which gives body,

30 - 75, preferably 40 - 60 % by weight of water, and optionally other additves such as antioxidants, aroma substances.

5. Formulation according to one of the preceding claims, characterized in that it contains decyl oleate as fatty alcohol oleate.

6. Formulation according to one of the preceding claims, characterized in that it contains sorbitan sesquioleate as sorbitan fatty acid ester.

7. Formulation according to one of the preceding claims, characterized in that it contains a mixture of higher saturated fatty alcohols with fatty alcohol sulphates and emulsifiers as the emulsifying additive which gives body

8. Formulation according to one of the preceding claims, containing:

8 - 12, preferably 10 % by weight isosorbide dinitrate, which, where in undissolved form, has an average particle size of 5 - 40, preferably 10 µm,

20 - 30, preferably 24 % by weight of decyl oleate,

1 - 5 preferably 2 % by weight of sorbitan sesquioleate,

5 - 7, preferably 6 % by weight of an emulsifying additive which gives body, consisting of a colloidal disperse mixture of cetylstearyl alcohol, sodium cetylstearyl sulphate and a triglyceride with 40 mol of ethylene oxide or fatty alcohol polyglycol ether,

50 - 60, preferably 58 % by weight of water, and other additives such as sorbic acid in amounts of less than 1 % by weight.

**Revendications**

1. Préparation pharmaceutique sous forme d'une pommade, contenant de l'isosorbide-dinitrate comme substance active sous la forme de fins cristaux au moins partiellement non dissous de même que des solvants organiques et de l'eau ainsi que, le cas échéant, un émulsionnant et d'autres additifs classiques, caractérisée en ce que le solvant organique possède un pouvoir dissolvant envers l'isosorbide-dinitrate inférieur à 5 % en poids, de préférence inférieur à 3 % en poids.

2. Préparation suivant la revendication 1, caractérisée en ce que le solvant organique consiste en un ou plusieurs des solvants du groupe des esters d'acides oléiques d'alcools gras et des esters non ionogènes d'acides gras du sorbitanne.

3. Préparation suivant la revendication 1 ou 2, caractérisée en ce que de l'isosorbide-dinitrate à l'état non dissous est présent en particules d'un diamètre inférieur à 100 µm, avec de préférence un diamètre moyen des particules de 5 à 40 µm.

4. Préparation suivant l'une des revendications précédentes, caractérisée en ce qu'elle contient:

5 à 15, de préférence 8 à 12 % en poids d'isosorbidedinitrate,

5 à 40, de préférence 20 à 30 % en poids d'un ester d'alcool gras d'acide oléique,

1 à 10, de préférence 1 à 5 % en poids d'un ester d'acide gras du sorbitanne,

1 à 8, de préférence 5 à 7 % en poids d'un additif émulsionnant et/ou donnant de la consistance,

30 à 75, de préférence 40 à 60 % en poids d'eau ainsi que, le cas échéant, d'autres additifs tels que des anti-oxydants, des arômes.

5. Préparation suivant l'une des revendications précédentes, caractérisée en ce qu'elle contient comme ester d'alcool gras de l'acide oléique, l'ester décylique de l'acide oléique.

6. Préparation suivant l'une des revendications précédentes, caractérisée en ce qu'elle contient du sesquioléate de sorbitanne comme ester d'acide gras du sorbitanne.

7. Préparation suivant l'une des revendications précédentes, caractérisée en ce qu'elle contient comme additif émulsionnant et donnant de la consistance un mélange d'alcools gras saturés supérieurs avec des sulfates d'alcools gras et des émulsionnants.

8. Préparation suivant l'une des revendications précédentes contenant:

8 à 12, de préférence 10 % en poids d'isosorbidedinitrate qui présente un diamètre moyen des particules de 5 à 40 µm, de préférence 10 µm, dans la mesure où il est présent sous la forme non dissoute;

20 à 30, de préférence 24 % en poids d'oléate de décyle,

1 à 5, de préférence 2 % en poids de sesquioléate de sorbitanne,

5 à 7, de préférence 6 % en poids d'un additif émulsionnant et donnant de la consistance, constitué d'un mélange en dispersion colloïdale d'alcool cétylstéarylique, de cétylstéarylsulfate de sodium et d'un triglycéride avec 40 moles d'oxyde d'éthylène ou d'éther polyglycolique d'alcool gras,

50 à 60, de préférence 58 % en poids d'eau, ainsi que d'autres additifs tels que l'acide sorbique, en quantités inférieures à 1 % en poids.